# FASCICULE DE BREVET EUROPEEN

(11) **EP 2 340 087 B1**
(45) Date de publication et mention de la délivrance du brevet: **06.07.2016**
(21) Numéro de dépôt: 09740467.7
(22) Date de dépôt: 18.08.2009
(51) Int. Cl.: A61N 7/02, A61B 8/08

(54) **DISPOSITIF DE TRAITEMENT THÉRAPEUTIQUE**
THERAPEUTISCHE BEHANDLUNGSVORRICHTUNG
DEVICE FOR THERAPEUTIC TREATMENT

(30) Priorité: 22.08.2008 FR 0855674
(43) Date de publication de la demande: 06.07.2011
(73) Titulaire: Theraclion SA, 92240 Malakoff (FR)
(72) Inventeur: LACOSTE, François, 75015 Paris (FR); PECHOUX, Thierry, 75019 Paris (FR)
(74) Mandataire: Hepp Wenger Ryffel AG
(86) Numéro de dépôt international: PCT/FR2009/051593
(87) Numéro de publication internationale: WO 2010/020730

(56) Documents cités:
- FR-A- 2 886 534
- US-A1- 2005 203 399
- US-A1- 2007 232 912
- US-A1- 2008 051 656
- US-B1- 6 508 774
- WU ET AL: "Using the Acoustic Interference Pattern to Locate the Focus of a High-Intensity Focused Ultrasound (HIFU) Transducer" ULTRASOUND IN MEDICINE AND BIOLOGY, NEW YORK, NY, US, vol. 34, no. 1, 1 janvier 2008 (2008-01-01), pages 137-146, XP022411276 ISSN: 0301-5629

## Description

La présente invention s'applique au domaine du traitement des êtres vivants par des dispositifs de thérapie ultrasonore.

En particulier, il s'agit de traitements par ultrasons focalisés (HIFU). Par conséquent, la présente invention concerne un dispositif de traitement thérapeutique, ainsi qu'un procédé de contrôle et d'optimisation de ce dispositif.

De manière classique dans la technologie HIFU (High Intensity Focused Ultrasound), un transducteur ultrasonore émet des ondes acoustiques de puissance concentrées dans une cible tissulaire. Ces ondes sont absorbées par le tissu, ce qui provoque une montée en température du tissu dans la zone focale et une coagulation de la cible.

Le traitement est généralement effectué sous contrôle échographique, avec une sonde échographique liée mécaniquement au transducteur, par exemple comme décrit dans FR 2 886 534. On obtient ainsi un système d'imagerie ultrasonore en ligne.

L'obtention de la coagulation du tissu dépend de plusieurs facteurs, comme par exemple :
- la puissance absorbée (elle-même fonction de l'absorption du tissu et de l'intensité acoustique *in situ*),
- la capacité calorifique du tissu, et
- des pertes thermiques (par conduction, par le flux sanguin).

Le transducteur peut être en contact direct avec le tissu, mais généralement on dispose un ballonnet rempli d'un gel ou d'un liquide capable de transmettre les ultrasons. Le ballonnet, grâce à sa souplesse, se conforme au tissu et permet de faciliter le contact acoustique entre le transducteur et le tissu. Généralement le liquide de couplage est réfrigéré, ce qui permet de protéger thermiquement le tissu au niveau de son interface avec le dispositif. Généralement encore, le liquide circule, permettant alors de maintenir la température de l'interface constante et d'évacuer les bulles éventuelles.

Un premier problème du traitement ultrasonore des tissus est le contrôle immédiat de l'efficacité du traitement des tissus exposés aux ultrasons, au niveau de la cible, mais aussi au niveau des tissus qu'il faut laisser intacts, tels que l'interface entre le dispositif et le tissu (la peau dans le cas d'un traitement extracorporel) ou d'autres structures anatomiques internes. Alternativement, on souhaiterait disposer d'un contrôle de la montée en température réelle du tissu. Une énergie ultrasonore insuffisante se traduit généralement par une cible peu ou mal coagulée, alors qu'une énergie trop forte met en danger les organes ou tissus environnant la cible. Or, les changements tissulaires induits par les ultrasons de puissance ne sont pas visibles avec l'échographie classique.

Comme il a été évoqué plus haut, la montée en température de la cible dépend de plusieurs facteurs :
- l'émission en puissance du transducteur,
- la transmission du faisceau vers la cible - et en particulier le couplage entre le transducteur et le tissu via par exemple la peau,
- l'absorption du tissu de la cible,
- la conduction thermique de la cible,
- la capacité calorifique et la chaleur de latence du tissu cible.

On comprend donc qu'une méthode permettant de contrôler l'effet dans la cible permettra de détecter un écart de l'un quelconque des facteurs ci-dessus et donc de résoudre l'un des problèmes techniques associés.

Or, il n'existe pas à ce jour de méthode satisfaisante pour la mesure non invasive et immédiate de la température à la cible ou des changements opérés dans le tissu. L'imagerie par résonance magnétique (IRM) permet de connaître la température du milieu, mais c'est une méthode lourde, coûteuse et le temps de réaction de la mesure est assez lent. Diverses méthodes de mesure de la température par ultrasons ont été proposées, mais aucune n'est satisfaisante, en raison principalement de leur grande sensibilité aux mouvements des patients.

Un deuxième problème est le contrôle de la bonne position du foyer (zone de concentration des ondes HIFU) par rapport à la cible.

Pour contrôler la position du foyer ou l'effet des ultrasons sur la cible, plusieurs auteurs recommandent l'observation par le système d'imagerie ultrasonore en ligne de taches hyperéchogènes dans la cible. Ces taches hyperéchogènes sont probablement créées par des gaz crées au foyer par les ultrasons de puissance, soit à cause de la cavitation induite par le champ acoustique, soit par le gaz dégagé par le tissu quand il est porté à haute température. On désigne ce phénomène par le terme « boiling ». L'observation des taches hyperéchogènes au foyer permet dans une certaine mesure de contrôler le traitement : en l'absence de tache hyperéchogène, on appliquera une énergie acoustique plus forte, alors qu'on diminuera l'énergie si on observe une tache hyperéchogène bien visible.

Or, pendant son émission, le faisceau de puissance provoque de fortes interférences ou perturbations sur l'image ultrasonore de la cible. Il se produit généralement un rideau d'interférence qui masque tout ou une partie de l'image, et en particulier la zone de la cible. Ce rideau d'interférence est superposé à l'image de repos (c'est-à-dire obtenue en l'absence de tir HIFU) du tissu, formant ainsi une image d'interférence. La structure de cette image d'interférence est de type aléatoire et elle se modifie au cours du tir. En pratique donc, on ne voit pas ou mal la cible pendant les tirs et l'observation des taches hyperéchogènes n'est possible qu'après interruption du faisceau de puissance (tir) sur l'image de repos. Dans l'art antérieur, on a proposé un système dans lequel les tirs et les observations sur les images de repos sont alternatifs, comme dans les documents US20080051656, US20060264748 et US20030028111. Cependant cette méthode requiert l'interruption du traitement pour pouvoir observer les images de repos, et notamment les taches hyperéchogènes.

Wu et al.: "Using the acoustic interference pattern to locate the focus of a high-intensity focused ultrasound (HIFU) transducer"; Ultrasound in Med. & Biol., Vol. 34; No. 1; 2008 et US 2007/0232912 A1 proposent un système pour localiser un point focal d'un transducteur acoustique avec une sonde d'imagerie.

A ce jour, il est donc pratiquement impossible de contrôler le tir pendant son émission avec des moyens non invasifs et peu coûteux.

La présente invention propose une solution aux problèmes susmentionnés en définissant un dispositif de traitement et un procédé de contrôle de la thérapie qui est non invasif, simple, peu coûteux, et ne requérant pas l'interruption du faisceau de puissance pendant la mesure.

Pour ce faire, la présente invention prévoit un dispositif de traitement thérapeutique comprenant un transducteur acoustique apte à émettre des ondes de puissance vers une cible pour la traiter, les ondes de puissance ayant un point focal, une sonde d'imagerie, telle qu'une sonde échographique, apte à émettre des ondes pour fournir une représentation imagée de la cible et de son environnement, avant, pendant et après l'émission des ondes de puissance issues du transducteur, la sonde et le transducteur étant solidaires l'un de l'autre, et, un dispositif d'affichage apte à afficher les images prélevées par la sonde, à savoir des images de repos prélevées avant et/ou après émission des ondes de puissance et des images d'interférence, prélevées pendant l'émission des ondes de puissance, le point focal des ondes de puissance étant situé dans l'image prélevée par la sonde, caractérisé en ce qu'il comprend en outre des moyens de détection pour détecter un changement de structure des images d'interférence qui est indicatif de l'effet et/ou de l'efficacité des ondes de puissance. Contrairement à l'art antérieur, on n'observe pas l'image de repos, prélevée avant ou entre les tirs, mais directement l'image d'interférence pendant les tirs. En cela, la présente invention surmonte un préjugé de l'art antérieur qui considère les images d'interférence comme des images perturbées ou parasitées qu'il est inutile ou impossible d'analyser. La présente invention va à l'encontre de la pratique courante qui consiste à observer les images de repos pour en déduire l'efficacité des tirs. Avec cette technique antérieure, les tirs sont bien souvent interrompus, non pas parce qu'ils ont atteint efficacement leurs cibles, mais pour observer sur les images de repos s'ils ont été suffisamment efficaces ou non. Ainsi, la séquence des tirs successifs n'est pas déterminée par l'efficacité des tirs, mais par la recherche de leur efficacité sur les images de repos, pour lesquelles il faut couper le tir. Avec la présente invention, l'effet et/ou l'efficacité du tir est contrôlé en temps réel directement à partir des images d'interférence en détectant un changement de structure représentatif de l'efficacité des tirs. En d'autres termes, l'invention consiste à contrôler les effets de l'énergie acoustique dans les tissus causés par les ultrasons de puissance en observant le parasitage de l'image échographique de contrôle.

Selon une caractéristique intéressante de l'invention, les moyens de détection peuvent comprendre des moyens de mesure de la brillance des images d'interférence. Ainsi, la structure des images d'interférence est analysée sur la base de leur brillance ou luminosité, et l'on va détecter un changement de structure des images d'interférence à partir d'un changement de brillance de ces images. Avantageusement, les moyens de mesure déterminent une zone de mesure qui est commune à plusieurs images d'interférence, et mesurent la brillance moyenne dans cette zone de mesure pour chaque image d'interférence. Cette zone de mesure peut être choisie comme formant tout ou partie de l'image d'interférence. De préférence, la zone de mesure forme une partie très réduite de l'image d'interférence, et elle est positionnée de manière à contenir le site anatomique que l'on veut observer, comme par exemple la cible ou un organe vital à proximité de la cible, mais qu'il ne faut pas endommager. La mesure de la brillance moyenne dans cette zone de mesure pour chacune des images d'interférence permet d'attribuer une valeur caractéristique à chaque image d'interférence, et l'on va observer un changement caractéristique de ces valeurs moyennes de brillance qui est indicatif de l'efficacité des ondes de puissance du tir.

Selon une forme de réalisation pratique, les images d'interférence sont des images composées par des niveaux de gris allant du blanc au noir, les moyens de mesure mesurant la moyenne des niveaux de gris dans la zone de mesure pour chaque image d'interférence. Cela est notamment le cas lorsque l'on utilise une sonde échographique qui délivre des images en noir et blanc constituées de niveaux de gris.

Selon une autre caractéristique intéressante de l'invention, les moyens de détection peuvent comprendre des moyens d'alerte aptes à déclencher un signal d'alerte lorsque les moyens de mesure mesurent un rehaussement subit de la brillance entre des images d'interférence successives. Ce rehaussement subit de la brillance constitue un changement de structure caractéristique des images d'interférence qui est indicatif de l'efficacité des ondes de puissance. On a pu remarquer de manière empirique que ce rehaussement subit de la brillance correspond avec l'apparition d'une tache hyperéchogène, observable sur les images de repos.

L'invention définit également un procédé de contrôle et d'optimisation d'un dispositif de traitement thérapeutique comprenant un transducteur acoustique apte à émettre des ondes de puissance vers une cible pour la traiter, une sonde d'imagerie telle qu'une sonde échographique, apte à émettre des ondes pour fournir une représentation imagée de la cible et de son environnement, avant, pendant et après l'émission des ondes de puissance issues du transducteur, et un dispositif d'affichage apte à afficher les images prélevées par la sonde, à savoir des images de repos, prélevées avant et/ou après émission des ondes de puissance et des images d'interférence, prélevées pendant l'émission des ondes de puissance, le procédé étant caractérisé en ce qu'il comprend de détecter un changement de structure des images d'interférence qui est indicatif de l'efficacité des ondes de puissance. Avantageusement, le procédé comprend les étapes suivantes : déterminer, pour un groupe d'images d'interférence successives dans le temps, une zone de mesure qui est commune aux images d'interférence du groupe, et mesurer la brillance de chacune des images d'interférence du groupe dans cette zone de mesure. Avantageusement, l'étape de mesure peut comprendre de mesurer la brillance moyenne dans la zone de mesure pour chacune des images d'interférence du groupe. Avantageusement, les images d'interférence sont des images composées par des niveaux de gris allant du blanc au noir, l'étape de mesure comprenant de mesurer la moyenne des niveaux de gris dans la zone de mesure pour chaque image d'interférence. De préférence, le procédé comprend en outre une autre étape consistant à détecter un rehaussement subit de la brillance mesurée entre des images d'interférence successives dans le temps.

L'esprit de la présente invention est de se servir des images d'interférence, et non pas images de repos, pour déterminer en temps réel l'efficacité des ondes de puissance du tir. La mesure de la brillance, notamment moyenne, dans une zone déterminée des images d'interférence, permet, grâce à une analyse comparative, de détecter tout changement de structure caractéristique et indicatif de l'efficacité du tir. Le paradoxe de la présente invention réside dans le fait que l'on analyse des images perturbées ou parasitées, alors que l'on dispose d'images nettes exemptes de perturbations ou de parasites.

L'invention sera maintenant décrite plus amplement en référence aux dessins joints donnant à titre d'exemple non limitatif un mode de réalisation de la l'invention.

Sur les figures :
- la figure 1 est une vue très schématique d'un dispositif de traitement thérapeutique selon l'invention,
- la figure 2 est une vue schématique illustrant le transducteur acoustique et la sonde d'imagerie lors du traitement de la tyroïde d'un patient,
- les figures 3a et 3b sont des représentations schématiques en négatif de deux images de repos, prélevées juste après une séquence de tirs,
- les figures 4a et 4b sont des vues schématiques en négatif d'images d'interférence prélevées respectivement juste avant les figures 3a et 3b,
- la figure 5 est un graphe représentant la brillance moyenne d'images d'interférence consécutives, et
- la figure 6 est un autre graphe représentant la relation linéaire qui existe entre le rehaussement de la brillance des images d'interférence et l'apparition de marques hyperéchogènes sur les images de repos.

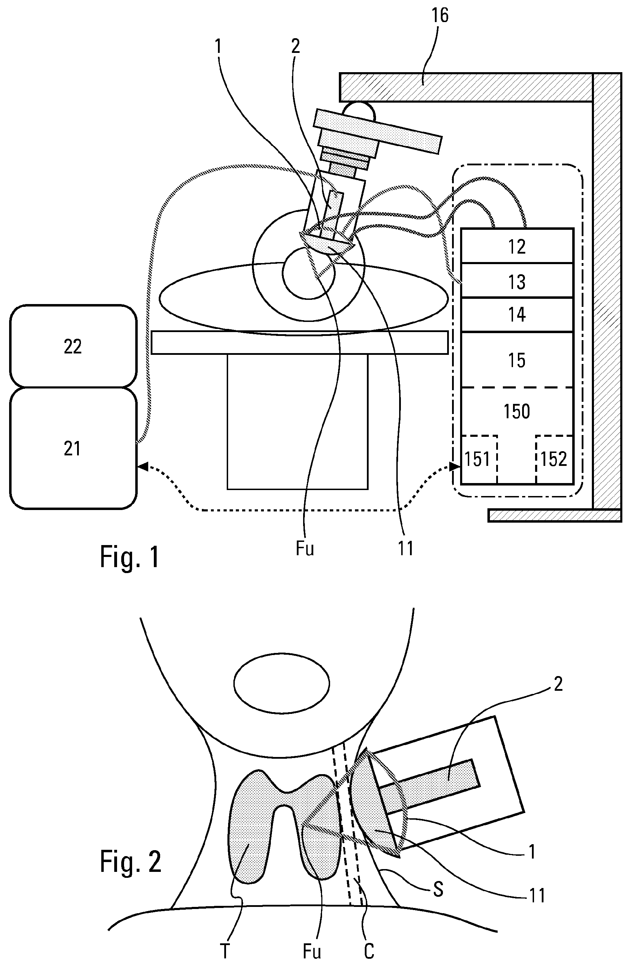

Il sera d'abord fait référence aux figures 1 et 2 pour décrire en détail les différents éléments constitutifs du dispositif de traitement thérapeutique de l'invention.

Le dispositif comprend tout d'abord une source d'émission acoustique 1 qui peut avantageusement être un transducteur ultrasonore adapté à produire un faisceau d'ultrasons Fu. De préférence, le transducteur ultrasonore est du type focalisé HIFU permettant de produire un faisceau focalisé d'ultrasons en un point focal précis. Le transducteur 1, comme on peut le voir sur la figure 2, peut comprendre une chambre remplie d'un fluide de couplage à travers lequel se propagent les faisceaux d'ultrasons. La chambre peut par exemple être délimitée par un ballonnet souple destiné à venir en contact intime avec une surface externe S d'une zone d'un corps où se situe une cible à traiter T. En général, la surface externe S est la peau du patient. Pour faire circuler le liquide de couplage à l'intérieur de la chambre 11, il est en général prévu des moyens de circulation 12 qui permettent de réguler le débit et la température du fluide de couplage à l'intérieur de la chambre 11. Le transducteur pour fonctionner a bien entendu besoin d'une alimentation de puissance 13 ainsi que d'une commande de déplacement 14 qui permet de déplacer et de localiser avec précision le transducteur par rapport au patient. Pour ce faire, le transducteur 1 est de préférence monté sur un bras articulé 16. Enfin, le transducteur est couplé à un ordinateur 15 qui permet de gérer tous les paramètres du transducteur, comme sa puissance, sa fréquence, sa durée d'impulsion, etc.

Le dispositif de traitement de l'invention comprend également des moyens d'imagerie qui peuvent par exemple se présenter sous la forme d'une sonde échographique 2 couplée à un échographe 21 et un écran de visualisation 22 qui affiche des vues ou images en coupe du site anatomique parcouru par la sonde 2. La sonde 2 peut être du type à barrettes. La sonde 2 est couplée mécaniquement au transducteur 1 comme on peut le voir sur les figures 1 et 2. Plus précisément, la sonde 2 et le transducteur 1 sont solidaires l'un de l'autre de sorte que la sonde 2 suit le point focal des faisceaux d'ultrasons Fu. La zone d'intensité maximale du faisceau d'ultrasons Fu est toujours représentée sur l'image de l'écran 22. Pour cela l'échographe 21 peut être couplé à l'ordinateur 15 du transducteur comme on peut le voir sur la figure 1.De préférence l'image échographique est acquise par l'ordinateur via la liaison électronique allant de 21 à 151.

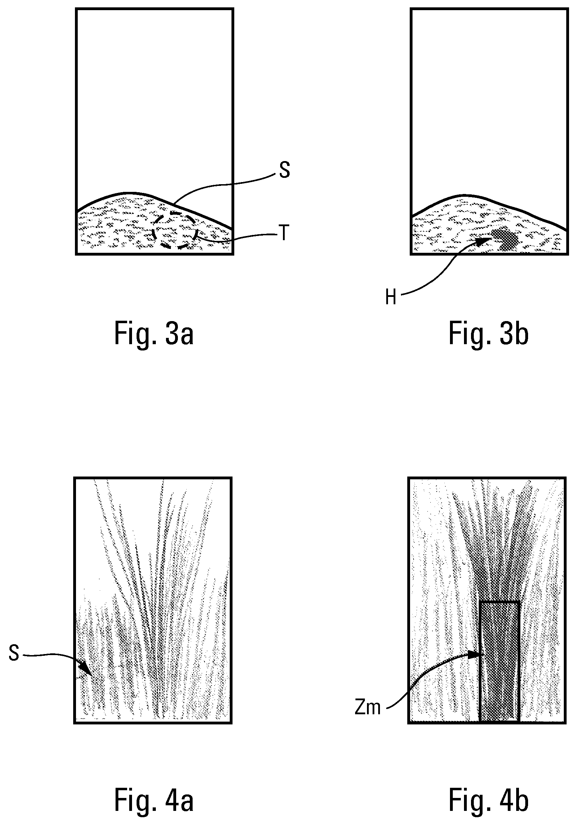

Avec une sonde échographique 2, l'échographe 21 produit sur l'écran de visualisation 22 des images successives dans le temps de la zone entourant le point focal des faisceaux d'ultrasons Fu. Ces images sont composées de pixels ayant des niveaux de gris allant du noir au blanc. Les pixels blancs sont représentatifs d'un élément très fortement échogène, alors que les pixels noirs sont représentatifs d'éléments très peu ou pas échogènes du tout. Ceci est bien connu pour les images échographiques. L'écran de visualisation 22 fournit des images avant l'émission des ondes de puissance Fu, pendant l'émission des ondes de puissance, et après l'émission des ondes de puissance. En d'autres termes, on dispose d'images échographiques avant, pendant et après les tirs. Les images prélevées avant et après les tirs sont relativement nettes et font apparaître la cible à traiter ainsi que son environnement anatomique. Ces images sont désignées ici sous le terme « d'images de repos », c'est-à-dire en l'absence de tirs. De telles images de repos sont représentées de manière schématique en négatif sur les figures 3 et 3b. Il s'agit en effet d'images en négatif, étant donné que les zones blanches sur ces figures apparaissent en réalité en noir sur l'écran de visualisation 22, et les zones noires apparaissent en blanc sur l'écran de visualisation. La partie basse des figures 3a et 3b représente le site anatomique tissulaire incorporant la cible T représentée en traits pointillés. On peut clairement identifier la peau S du patient. La figure 3b fait clairement apparaître une marque hyperéchogène H. Outre ces images de repos, l'écran de visualisation produit également des images correspondant aux phases de tirs. Du fait de la puissance apportée par les ondes de puissance Fu, des interférences considérables sont générées qui perturbent ou parasitent l'image, de sorte que la représentation du site anatomique tel que visible sur les figures 3a et 3b n'est plus ou que très faiblement perceptible. On peut dire que les interférences produites par les ondes de puissance se superposent à l'image de repos, mais ces interférences sont tellement fortes que l'image de repos est très fortement dégradée. On désignera ci-après les images durant les phases de tirs sous le terme « d'images d'interférence ». De telles images sont représentées de manière schématique en négatif sur les figures 4a et 4b. La figure 4a a par exemple été prélevée juste avant l'image de repos 3a. Quant à l'image d'interférence de la figure 4b, elle a par exemple été prélevée juste avant l'image de repos de la figure 3b. On peut très difficilement discerner sur la figure 4a le contour de la peau S. Les interférences qui se présentent sous la forme de faisceaux divergents vers le haut masquent presque entièrement la représentation du site anatomique, tel que visible sur les figures 3a et 3b. Ceci est encore plus marquant sur la figure 4b où les interférences sont particulièrement fortes, de sorte que le site anatomique n'est plus visible du tout. C'est la raison pour laquelle ces images d'interférence n'ont jusqu'à présent jamais été utilisées pour en tirer une quelconque information quant à la présence et/ou l'efficacité des ondes de puissance du tir.

Or, on a observé de manière inattendue que lorsqu'il se produit une tache hyperéchogène H au foyer, l'image d'interférence est fortement modifiée. Ceci est représenté sur les figures 3b et 4b, qu'il faut comparer aux figures 3a et 4a. Pour rappel, les taches hyperéchogènes sont attribuées à la formation supposée de gaz au foyer, aussi connue sous le terme de « boiling ». On a notamment observé un rehaussement de la brillance ou luminosité de l'image d'interférence. On peut également observer une modification de la structure de l'image : le rehaussement de sa brillance est plus important en regard des tissus dont la brillance (sur l'imagerie ultrasonore) augmente sous l'effet de la thérapie. C'est ainsi que la brillance de l'image d'interférence (figure 4b) est renforcée dans la zone de l'image située autour du foyer lorsque précisément une tache hyperéchogène apparaît dans cette même zone focale. L'intérêt de cette observation est que pendant les tirs, cette tache hyperéchogène est cachée par l'image d'interférence et ne peut donc pas être observée directement, alors que les modifications de l'image d'interférence sont bien visibles.

La figure 4b montre comment il est possible de détecter un rechaussement de la brillance de l'image d'interférence dans une zone de mesure prédéfinie de l'image, qui est désignée Zm. On mesure la brillance de l'image, c'est à dire le niveau de gris, dans la zone Zm. De préférence cette zone Zm englobe le foyer du transducteur de tir.

La zone est déterminée de manière à prendre en compte préférentiellement la partie de l'image qui se rehausse le plus lors d'un tir qui provoque une marque hyperéchogène. On évite de prendre en compte toute l'image d'interférence, parce qu'on constate parfois des rehaussements dans les parties latérales de l'image, qui ne sont pas indicatifs d'un traitement efficace. Typiquement, la zone de mesure est de forme allongée vers le transducteur, est centrée latéralement sur le point focal, englobe celui-ci, mais est décalée vers le transducteur de manière à englober une partie de la zone pré-focale.

On peut cependant définir une zone Zm qui n'inclut pas la zone focale, par exemple englobant une structure anatomique qu'on veut protéger des ondes acoustiques. En effet, on constate qu'une structure adjacente à la cible peut devenir hyperéchogène au cours du traitement, ce qui indique qu'elle est modifiée par les ultrasons. Ce peut être le cas de la peau ou des tissus sous-cutanés, comme la carotide C visible sur la figure 2. Cet effet doit en général être évité et cela est possible grâce à l'invention. Selon l'invention, on placera une zone de mesure Zm autour de l'image la zone anatomique à protéger et on surveillera le rehaussement éventuel de l'image d'interférence dans cette zone.

On peut calculer la moyenne de brillance des niveaux de gris dans la zone Zm prise pendant le tir: cette valeur permet tout d'abord de détecter la présence d'une image d'interférence et donc de contrôler l'émission effective des tirs de puissance. La valeur calculée permet aussi de détecter un éventuel rehaussement des niveaux de gris lors d'un tir efficace. Ce rehaussement se produit notamment si l'interférence se concentre dans la zone Zm. Or, ce rehaussement de la brillance, qui s'avère lié à la présence d'une marque hyperéchogène, révèle d'un changement de structure de l'image d'interférence qui est caractéristique ou indicatif de l'effet et/ou de l'efficacité du tir.

Plusieurs méthodes sont possibles pour détecter l'efficacité du tir HIFU, Dans un premier mode, on fait la moyenne pixel à pixel dans la zone Zm de chaque image prise pendant le tir. Chacune des moyennes obtenues sur une image sont ensuite moyennées dans le temps pendant la durée du tir. Si le chiffre obtenu est supérieur à un seuil expérimentalement fixé, le tir HIFU est considéré comme efficace.

Dans un deuxième mode, on fait également la moyenne pixel à pixel dans la zone Zm de l'image à un instant t du tir, à laquelle on soustrait la moyenne pixel à pixel dans la zone Zm de l'image à un instant précédent t-dt. Si le chiffre obtenu est supérieur à un seuil expérimentalement fixé, le tir HIFU est considéré comme efficace. Ce deuxième mode permet d'interrompre le tir lorsque le seuil d'efficacité est atteint.

Pour détecter une marque hyperéchogène, on fait la différence pixel à pixel dans la zone Zm d'une image prise après le tir avec une image prise avant le tir. On calcule ensuite le maximum de cette différence. Ce maximum peut être utilisé comme indicateur d'absence ou de présence d'une marque hyperéchogène.

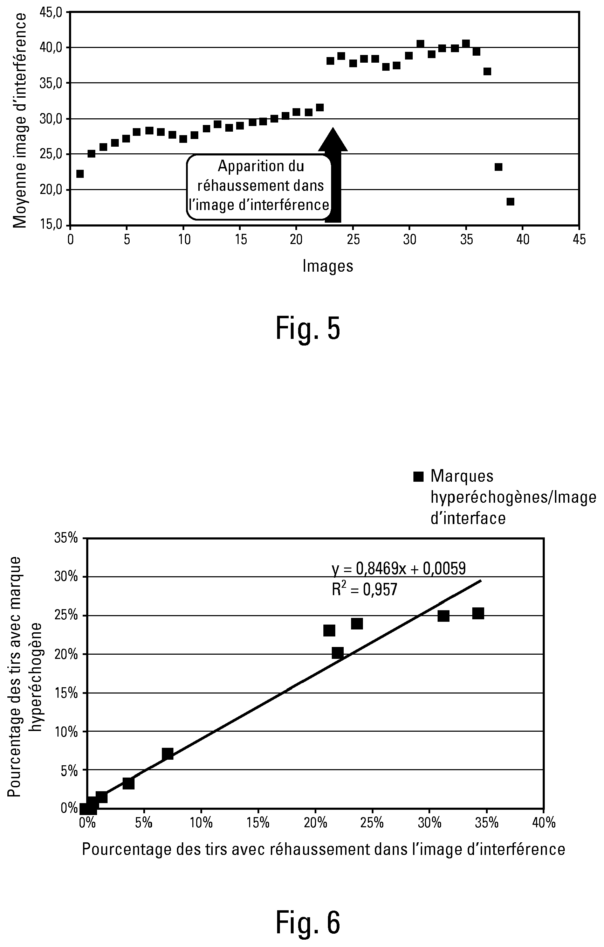

La figure 5 montre l'évolution du niveau de gris moyen dans la zone Zm au fur et à mesure de la progression d'un tir, selon le deuxième mode. On peut remarquer que la courbe présente un décrochage brutal vers le haut qui correspond à un rehaussement subit de la brillance, indicatif de la présence d'une tache hyperéchogène, et donc d'un traitement efficace.

La figure 6 montre des résultats, obtenus chez 11 patients, porteurs d'un nodule thyroïdien et traité par HIFU. On a analysé a posteriori les images échographiques enregistrées en vidéo pendant les tirs. On a détecté après chaque tir la présence ou l'absence de marque hyperéchogène, et pendant chaque tir le rehaussement subit éventuel de l'image d'interférence. Ces détections ont été faites avec les méthodes d'analyse d'image spécifiées plus haut. On voit qu'il y a une très bonne corrélation entre les deux types de détection.

Par ailleurs, on a également constaté une corrélation, pratiquement linéaire, entre la réussite du traitement et le pourcentage de tirs pendant lesquels on détecte un rehaussement subit de l'image d'interférence.

Il s'agit des mêmes traitements de nodules thyroïdiens par HIFU qu'au paragraphe précédent. Le but des traitements étant de réduire le volume de ces nodules, la réussite du traitement est jaugée par le pourcentage de réduction de ces nodules.

La mesure du rehaussement subit de l'image d'interférence est donc prédictive de la réussite du traitement.

Afin d'analyser les images d'interférence de la manière décrite ci-dessus, et notamment pour détecter le changement de structure caractéristique des images d'interférence, qui est indicatif de l'efficacité des ondes de puissance, le dispositif de traitement thérapeutique intègre des moyens de détection 150, qui peuvent par exemple être intégrés à l'ordinateur 15. Ces moyens de détection peuvent se présenter sous la forme d'un logiciel de traitement approprié. Les moyens de détection 150 comprennent entre autre des moyens de mesure de la brillance ou luminosité 151 des images d'interférence. Avantageusement, ces moyens d'interférence 151 permettent de déterminer le positionnement et la forme de la zone de mesure Zm dans laquelle la brillance moyenne pour chaque image d'interférence va être mesurée. Les moyens de mesure 151 vont notamment mesurer la moyenne des niveaux de gris dans la zone de mesure Zm pour chaque image d'interférence. Les moyens de détection 150 peuvent également comprendre des moyens d'alerte 152 aptes à déclencher un signal d'alerte lorsque les moyens de mesure 151 détectent un rehaussement subit de la brillance entre deux images d'interférence consécutives. Le signal d'alerte peut être sonore ou visuel, par exemple affiché sur l'écran de l'ordinateur ou sur l'écran de visualisation 22. Le signal d'alerte peut être aussi couplé à une commande de l'alimentation de puissance 13 afin d'interrompre ou de moduler la puissance ultrasonore émise par le transducteur 1.

Grâce à l'invention, on peut surveiller en temps réel la présence et plus particulièrement l'efficacité des ondes de puissance émises, sans avoir à interrompre le tir.

## Revendications

1. Dispositif de traitement thérapeutique comprenant :
- un transducteur acoustique (1) apte à émettre des ondes de puissance (Fu) vers une cible (T) pour la traiter, les ondes de puissance ayant un point focal,
- une sonde d'imagerie (2), telle qu'une sonde échographique, apte à émettre des ondes pour fournir une représentation imagée de la cible (T) et de son environnement, avant, pendant et après l'émission des ondes de puissance (Fu) issues du transducteur (1),
- un dispositif d'affichage (21, 22) apte à afficher les images prélevées par la sonde (2), à savoir des images de repos prélevées avant et/ou après émission des ondes de puissance (Fu) et des images d'interférence, prélevées pendant l'émission des ondes de puissance (Fu), le point focal des ondes de puissance étant situé dans l'image prélevée par la sonde et des moyens de détection (150) pour détecter un changement de structure des images d'interférence qui est indicatif de l'effet des ondes de puissance (Fu)
**caractérisé en ce que** la sonde et le transducteur sont solidaires l'un de l'autre, de sorte que la sonde suit le point focal des ondes de puissance (Fu).

2. Dispositif de traitement thérapeutique selon la revendication 1, dans lequel les moyens de détection (150) comprennent des moyens de mesure de la brillance (151) des images d'interférence.

3. Dispositif de traitement thérapeutique selon la revendication 2, dans lequel les moyens de mesure (151) sont aptes à déterminer une zone de mesure (Zm) qui est commune à plusieurs images d'interférence, et à mesurer la brillance moyenne dans cette zone de mesure (Zm) pour chaque image d'interférence.

4. Dispositif de traitement thérapeutique selon la revendication 3, dans lequel les images d'interférence sont des images composées par des niveaux de gris allant du blanc au noir, les moyens de mesure (151) étant aptes à mesurer la moyenne des niveaux de gris dans la zone de mesure (Zm) pour chaque image d'interférence.

5. Dispositif de traitement thérapeutique selon la revendication 2, 3 ou 4, dans lequel les moyens de détection (150) comprennent des moyens d'alerte (152) aptes à déclencher un signal d'alerte lorsque les moyens de mesure (151) mesurent un rehaussement subit de la brillance entre des images d'interférence successives, le signal d'alerte étant avantageusement couplé au transducteur (1) afin de l'interrompre ou de moduler sa puissance.

## Patentansprüche

1. Therapeutische Behandlungsvorrichtung, umfassend:
- einen akustischen Wandler (1), der geeignet ist, Leistungswellen (Fu) zu einem Ziel (T) zu dessen Behandlung zu senden, wobei die Leistungswellen einen Brennpunkt haben,
- eine Bildgebungssonde (2), wie eine echographische Sonde, die geeignet ist, Wellen zu entsenden, um eine bildhafte Darstellung des Ziels (T) und seiner Umgebung vor, während und nach dem Senden der vom Wandler (1) ausgehenden Leistungswellen (Fu) zu liefern,
- eine Anzeigevorrichtung (21, 22), die geeignet ist, die durch die Sonde (2) aufgenommenen Bilder, nämlich Bilder einer Ruhestellung, die vor und/oder nach dem Senden der Leistungswellen (Fu) aufgenommen werden, und Interferenzbilder anzuzeigen, die während des Sendens der Leistungswellen (Fu) aufgenommen werden, wobei sich der Brennpunkt der Leistungswellen in dem Bild befindet, das von der Sonde und von Erkennungsmitteln (150) zum Erkennen einer Strukturänderung der Interferenzbilder, die einen Hinweis auf die Wirkung der Leistungswellen (Fu) gibt, aufgenommen wurde,
**dadurch gekennzeichnet, dass** die Sonde und der Wandler miteinander verbunden sind, so dass die Sonde dem Brennpunkt der Leistungswellen (Fu) folgt.

2. Therapeutische Behandlungsvorrichtung nach Anspruch 1, bei der die Erkennungsmittel (150) Mittel zum Messen der Brillanz (151) der Interferenzbilder umfassen.

3. Therapeutische Behandlungsvorrichtung nach Anspruch 2, bei der die Mittel (151) zum Messen geeignet sind, eine Messzone (Zm) zu bestimmen, die mehreren Interferenzbildern gemein ist, und die durchschnittliche Brillanz in dieser Messzone (Zm) für jedes Interferenzbild zu messen.

4. Therapeutische Behandlungsvorrichtung nach Anspruch 3, bei der die Interferenzbilder Bilder sind, die sich aus Graustufen von weiß bis schwarz zusammensetzen, wobei die Mittel (151) zum Messen geeignet sind, den Durchschnitt der Graustufen in der Messzone (Zm) für jedes Interferenzbild zu messen.

5. Therapeutische Behandlungsvorrichtung nach Anspruch 2, 3 oder 4, bei der die Erkennungsmittel (150) Alarmmittel (152) umfassen, die geeignet sind, ein Alarmsignal auszulösen, wenn die Mittel (151) zum Messen eine plötzliche Erhöhung der Brillanz zwischen aufeinanderfolgenden Interferenzbildern messen, wobei das Alarmsignal vorteilhafterweise mit dem Wandler (1) gekoppelt ist, um ihn zu unterbrechen oder seine Leistung zu modulieren.

## Claims

1. Device for therapeutic treatment comprising:
- an acoustic transducer (1) able to emit power waves (Fu) toward a target (T) in order to treat it, the power waves having a focal point,
- an imaging probe (2), such as an echography probe, able to emit waves to provide an imaged representation of the target (T) and of its environment, before, during and after the emission of the power waves (Fu) from the transducer (1),
- a display device (21, 22) able to display the images taken by the probe (2), namely images at rest taken before and/or after the emission of the power waves (Fu) and interference images, taken during the emission of the power waves (Fu), the focal point of the power waves being situated in the image taken by the probe, and detection means (150) for detecting a change of structure of the interference images which is indicative of the effect of the power waves (Fu),
**characterized in that** the probe and the transducer are integrally linked to one another, so that the probe follows the focal point of the power waves (Fu).

2. Device for therapeutic treatment according to Claim 1, in which the detection means (150) include means (151) for measuring the brightness of the interference images.

3. Device for therapeutic treatment according to Claim 2, in which the measurement means (151) are able to determine a measurement area (Zm) which is common to a number of interference images, and to measure the average brightness in this measurement area (Zm) for each interference image.

4. Device for therapeutic treatment according to Claim 3, in which the interference images are images composed of gray levels ranging from white to black, the measurement means (151) being able to measure the average of the gray levels in the measurement area (Zm) for each interference image.

5. Device for therapeutic treatment according to Claim 2, 3 or 4, in which the detection means (150) include warning means (152) able to trigger a warning signal when the measurement means (151) measure a sudden rise in brightness between successive interference images, the warning signal advantageously being coupled to the transducer (1) in order to stop it or modulate its power.
